(19) Europäisches Patentamt
European Patent Office
Office européen des brevets

(11) EP 2 665 083 A2

(12) **EUROPEAN PATENT APPLICATION**

(43) Date of publication:
20.11.2013 Bulletin 2013/47

(51) Int Cl.:
*H01J 37/22* (2006.01)     *H01J 37/26* (2006.01)
*H01J 37/28* (2006.01)

(21) Application number: 13167952.4

(22) Date of filing: 16.05.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 17.05.2012 US 201213474176

(71) Applicant: FEI COMPANY
Hillsboro, Oregon 97124-5793 (US)

(72) Inventors:
• Bugge, Cliff
Portland, OR Oregon 97230 (US)
• Van Leer, Brandon
Cornelius, OR Oregon 97113 (US)

(74) Representative: Bakker, Hendrik
FEI Company
Patent Department
P.O. Box 1745
5602 BS Eindhoven (NL)

(54) **Scanning microscope having an adaptive scan**

(57) A method of using a scanning microscope to rapidly form a digital image of an area. The method includes performing an initial set of scans to form a guide pixel set for the area and using the guide pixel set to identify regions representing structures of interest in the area. Then, performing additional scans of the regions representing structures of interest, to gather further data to further evaluate pixels in the regions, and not scanning elsewhere in the area.

**FIG. 7**

EP 2 665 083 A2

Description

## Technical Field of the Invention

The present invention relates to a method and an apparatus incorporating the method for adaptive scanning in a scanning microscope.

## Background of the Invention

Forming a high resolution digital image of a volume by a scanning electron microscope (SEM)/focused ion beam (FIB) dual beam device is an increasingly useful tool in the fields of biology (examination of tissue) and natural resource exploitation (examination of core samples). In this technique the FIB iteratively slices the volume to be imaged, thereby progressively exposing surfaces and the SEM makes an image of each exposed surface. This process may entail the collection of an immense amount of data, typically in the giga pixel range. This data collection can be very time-consuming, taking anywhere from 4 to 60 hours. Having to wait such a long period of time can represent a major roadblock to researchers attempting to learn more about the nature of a problem as quickly as possible. Moreover, it limits the throughput of a device.

## Summary of the Invention

Embodiments of a method and apparatus are provided herein for scanning a specimen to form digital imagery, with a set of guide data initially gathered and used to eliminate from further scanning regions of the specimen that are determined to be not of interest. The elimination from scanning of the regions that are not of interest speeds up scanning, making the process more efficient.

In a first separate aspect, the present invention may take the form of a method of using a scanning microscope to rapidly form a digital image of an area. The method includes performing an initial set of scans to form a guide pixel set for the area and using the guide pixel set to identify regions representing structures of interest in the area. Then, performing additional scans of the regions representing structures of interest, to gather further data to further evaluate pixels in the regions, and not scanning elsewhere in the area.

In a second separate aspect, the present invention may take the form of a scanning microscope assembly, adapted to perform a scan of an area, and as a part of performing the scan of the area, adapted to perform an initial set of scans to form a guide pixel set for the area and then to use the guide pixel set to identify regions representing structures of interest in the area. Then, the microscope performs additional scans of the regions representing structures of interest, to gather further data to further evaluate pixels in the regions, and does not scan elsewhere in the area.

In a third separate aspect, the present invention may take the form of a method of forming an image of an area containing regions of interest and regions not of interest using a scanning imaging device. The method includes performing a brief scan, thereby collecting less accurate information than possible, but also taking less time and using data from the brief scan to determine pixels representing regions not of interest. Then, performing a scan at a slower rate, but not scanning pixels determined to represent regions not of interest, thereby completing the scan more quickly.

In a fourth separate aspect, the present invention may take the form of a scanning electron microscope assembly including a data entry sub-assembly, adapted to permit a user to input dimensional characteristics of structures of interest in a specimen to be scanned, and wherein the dimensional characteristics affect scanning of the specimen.

The foregoing has outlined rather broadly the features and technical advantages of the present invention in order that the detailed description of the invention that follows may be better understood. Additional features and advantages of the invention will be described hereinafter. It should be appreciated by those skilled in the art that the conception and specific embodiments disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims.

## Brief Description of the Drawings

For a more thorough understanding of the present invention, and advantages thereof, reference is now made to the following descriptions taken in conjunction with the accompanying drawings, in which:

FIG. 1 shows a micrograph of yeast cells set in epoxy;

FIG. 2 shows a micrograph of shale interspersed with other mineral substance;

FIG. 3 shows a probability density function of an actual pixel grayscale, given a pixel sample grayscale value, and a threshold set to yield a low probability of falsely finding a low pixel grayscale;

FIG. 4 shows a probability density function of an actual pixel grayscale, given an averaged set of pixel sample grayscale values and a threshold set to yield a low probability of falsely finding a low pixel grayscale;

FIG. 5 shows a probability density function of an actual pixel grayscale, given an averaged set of pixel sample grayscale values, wherein the set contains more sample values than the set of FIG. 4, and a threshold set to yield a low probability of falsely finding a low pixel grayscale;

FIG. 6 shows a set of four yeast cells, set in a solid medium and shown in cross-section; and

FIG. 7 shows a top view of a yeast cell, having a transparent outer membrane so that the cell organs are visible.

FIG. 8 shows a side-section view of the yeast cell of FIG. 7, taken along line 8-8 of FIG. 7.

FIG. 9 shows a side-section view of the yeast cell of FIG. 7, taken along line 9-9 of FIG. 7.

FIG. 10 shows an exemplary dual beam FIB/SEM system that could be used to implement preferred embodiments of the present invention.

**Detailed Description of Preferred Embodiments**

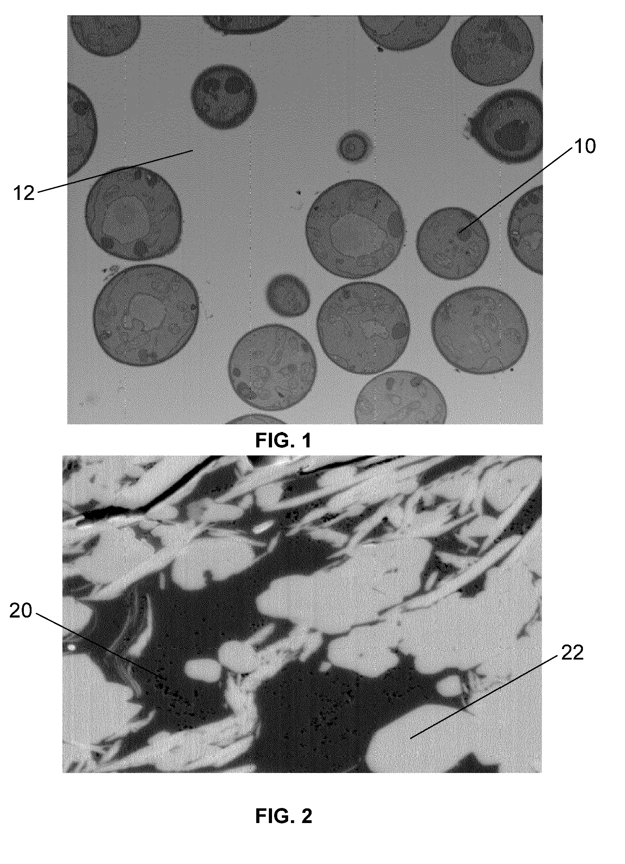

Embodiments of the present invention are directed to methods and apparatuses incorporating methods that reduce the number of data samples that must be acquired in forming an image of a volume. One preferred embodiment relies on the fact that typically in an imaged volume there may be specific portions, representing structures of interest, with the rest of the image data being of little or no value. For example, in FIG. 1, the cells 10 are structures of interest whereas the epoxy mounting medium 12, appearing as a field of light gray in the image is not of interest. In like manner, in FIG. 2 the shale 20 is of interest, whereas the interstitial material 22 which appears as a field of white is not of interest.

In order to avoid scanning, to collect pixel data, in areas that are not of interest a determination must be made, by the data processor that typically forms a part of a scanning image formation device, as to what areas represent structures of interest, and what areas do not, prior to fully scanning an area. In the two examples given by FIGS. 1 and 2, the regions that are not of interest are lighter than the regions of interest. When this is the case, thresholding for darker gray shades and eliminating pixels that are not dark enough (high enough return level) represents one method of discriminating regions of interest from those that are not of interest.

To obtain a preliminary estimate of gray shade value, sufficient to decide whether a pixel is worth further scanning, three techniques may be used, all of which are described in greater detail in the following paragraphs. First, in a system in which a sequence of scans are added together to form an integrated scan, each pixel grayscale in a first scan or a first set of scans can be compared to a threshold to make the determination, which is then applied to subsequent scans of the integrated scan. Second, in the imaging of a volume by an FIB/SEM dual beam system, previously imaged surfaces may have areas that are within the same structure of interest. A biological structure, for example, may extend through many of the progressively revealed surfaces. Accordingly, a determination may be made as to the location of structures of interest in the previous surface, and this information may be used, together with the initial scan data of an integrated scan, to find these structures of interest in surface currently being imaged. Finally, within a single scan, previously scanned pixels may be correlated with prospectively scanned pixels.

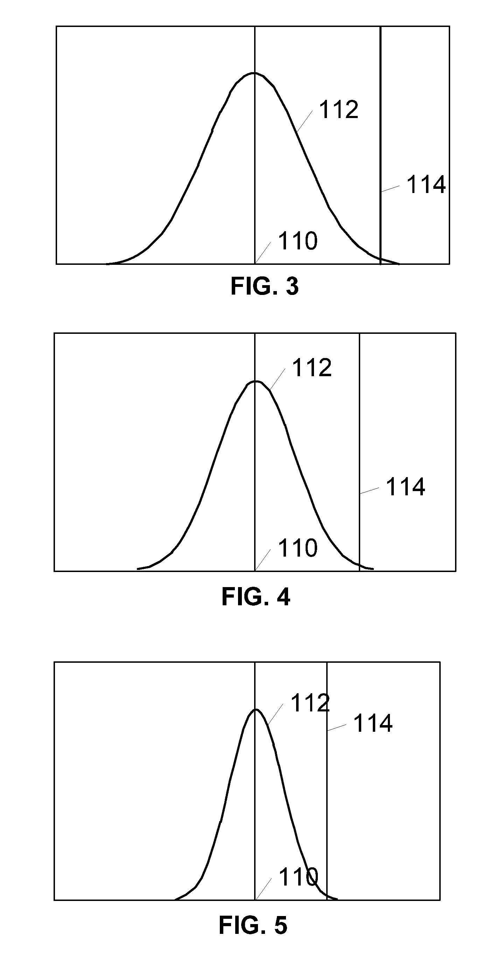

As noted, a scanning electron microscope forms an image by scanning a specimen area multiple (e. g. 20) times with an electron beam while secondary electrons are collected by a secondary electron detector to form an image of the specimen area. This is done to prevent a buildup of charge in any specimen location as the beam dwells at the location. For each scan, there is a measurement of the responsiveness to the beam, that is, the number of secondary electrons collected, for each pixel (that is, a measurement of true pixel grayscale). For the initial scans, these pixel grayscale measurements can be compared to a threshold, to determine if some are of a low enough value that they can be fairly presumed to be from a region that is not of interest. But, referring to FIG. 3, after a single scan (from a series of scans) each such measurement 110 (of a measured value that is exactly at a true pixel grayscale theoretical threshold), has a fairly wide error probability density function (PDF) 112, indicating that it is fairly likely that the actual pixel grayscale is considerably higher than the pixel grayscale measurement. This requires that a conservative threshold 114 be set to avoid falsely deciding to not scan the pixel in the future scans. Referring to FIG. 4, by integrating the scans, for each integrated pixel measurement 110, the error PDF 112 is narrowed and the threshold 114 can be reduced, thereby eliminating more pixels from future scanning. After still more scanning, the PDF is reduced in width still further, as shown in FIG. 5, and a still tighter threshold 114 may be used without too great a chance of excluding a pixel that is actually more intense than indicated by initial scanning.

In a preferred embodiment, this process is continued until either a predetermined number of scans have been performed, or the number of additional pixels excluded by the process falls below a predetermined level. The end of pixel elimination is not necessarily the end of scanning, however, because it may be desirable to continue to collect data to more precisely evaluate pixel grayscales in the regions of interest. In one preferred embodiment, a region having a particularly variegated scan response is scanned an additional number of times, beyond the standard number of scans in an integrated scan, so that even more precise pixel grayscales may be formed in that region. In an alternative preferred embodiment pixels are eliminated from future scanning only after an initial scan, of the integrated scanning process. In an additional alternative preferred embodiment closed forms are detected and included in future scanning, while pixels not inside a closed form are eliminated from future scanning.

In a focused ion beam (FIB)/scanning electron microscope (SEM), the FIB cuts away a partial layer of material and an SEM images the exposed surface. After the initial surface has been imaged, it can be used as a guide, giving a preliminary indication of the location of structures of interest in the immediately subsequent scan. Many specimens include structures of interest that are regularly shaped (for example cells, in a biological specimen) and are larger than the spacing between imaged planes. Accordingly, a pixel occurring outside of a structure of interest in a first imaged plane yields a good indication that the same will be true of a corresponding pixel in a neighboring plane. This information is merged with the information from the first scan or scans from the integrated scanning, to provide an enhanced indicator for whether or not a pixel should be included in a subsequent scan.

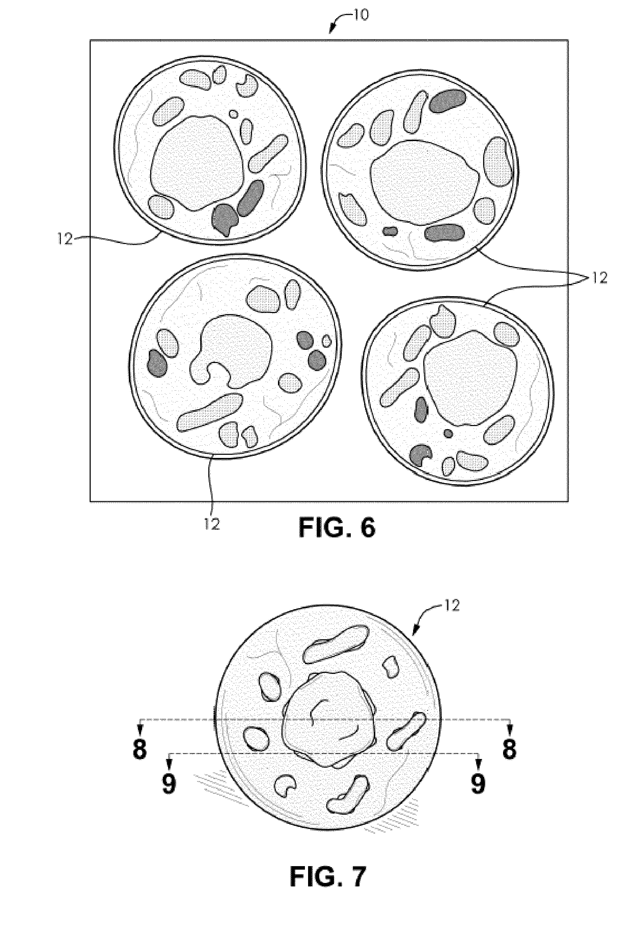

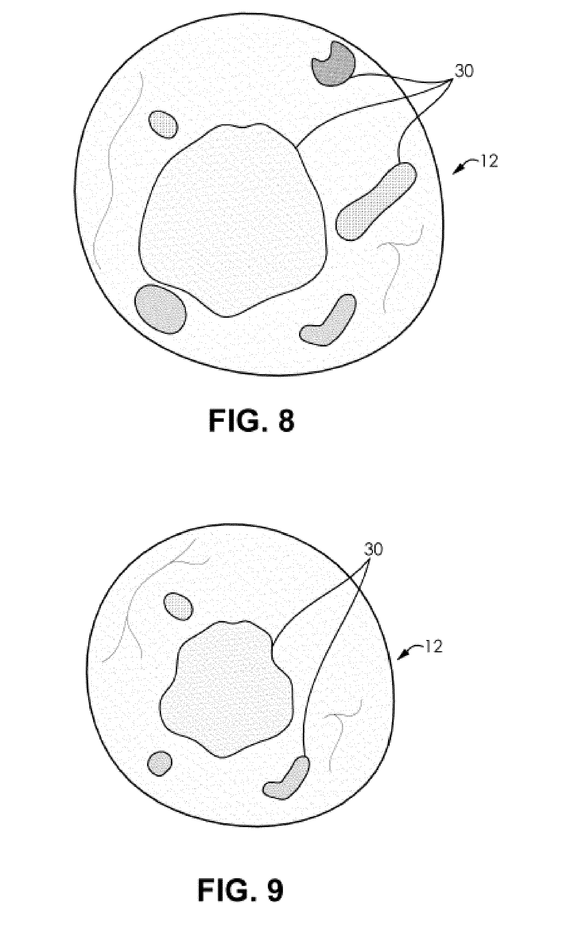

FIGS. 6-9 illustrate this point, with FIG. 6 showing a set of four yeast cells set into epoxy, FIG. 7 showing a top view of the upper left one of these cells, and FIGS. 8 and 9 showing closely spaced sections of the cell of FIG. 7, taken along planes 8-8 and 9-9 defined in FIG. 7. A preferred embodiment relies on a correlation between data from a first image plane as illustrated in FIG. 8, and a second image plane as shown in FIGS. 9, for the cell 12. As can be seen, there is a general similarity between the two slices, due to their close proximity. This similarity can be harvested by correlating pixels from the cut of FIG. 8, with the prospective evaluation of nearby pixels in the next frame (FIG. 9). The first level effort is to aid in the determination of which pixels represent mere epoxy, as opposed to cell matter 12, and to avoid scanning those areas past the first scan of an integrated scan. In a preferred embodiment, this correlation factor is continued for pixels near the margins of cell 12 as these are the areas have a greater likelihood of not reflecting the region-of-interest value (true or false) of the similarly positioned pixel in the previously scanned plane. In a preferred embodiment pixels that are within the cell boundary are given a very high correlation (0.85) for region-of-interest value in the next plane, and this value declines linearly until a distance of twenty pixels from the cell boundary is reached. In a more advanced embodiment, pixel grayscales may be used to gain a faster evaluation of pixel grayscales in the next scan, for example when a cell organ 30 appears in both scans and is generally the same material.

Additionally, after the beginning of a scan, information from neighboring pixels is correlated with any pixel about-to-be scanned and can be used to increase the certainty of the thresholding to determine if the pixel should be scanned. In a preferred embodiment, to make this decision, a scan other than a straight raster scan is made. For example, a spaced [[a ]]scan of every tenth row and column of pixels may help determine where the regions of interest are, before performing more detailed scans.

Also, the pixel variance in an area about the pixel of interest, either from an initial scan or set of scans in an integrated scan process, or from previously scanned pixels within the same scan, may be used to determine the presence of a region of interest. This is because a region of interest will tend to have more features than does a background area, such as a volume of undifferentiated epoxy.

Putting together all of the above discussed factors, in a preferred embodiment, used in the scanning of a volume in which regions of interest have higher value pixels than regions that are not of interest and in which areas with higher pixel variance are more likely to represent regions of interest than regions with lower pixel variance, the following thresholding inequality is used to determine which pixels to scan:

$$T < C_R*R + C_{PSF}*(\text{Prev Surface Factor}) + * \Sigma C_{SP}(SP.Px-R)$$
$$+ C_V*(\text{Regional Pixel Variance}) - \varepsilon$$
$$(1)$$

Where:

T = Threshold

$C_R$ = Weighting constant for same pixel in previous scans, of an integrated scan

R= Reading of pixel beam responsiveness in previous scans

$C_{PSF}$ = Weighting constant for Previous Surface Factor

Prev Surface Factor = 1 if pixel is within a region of interest in the neighboring previously scanned surface; = 1/#ofpix, where #ofpix = number of pixels separated from a region of interest in the neighboring previously scanned plane, for #ofpix < 20; and = 0 otherwise.

$C_{SP}$ = Weighting constant for each correlated same scan, same plane, already scanned pixels

SP.Px = correlated, already scanned, same plane pixels

$C_V$ = Weighting constant for Regional Pixel Variance

Regional Pixel Variance = Variance of Pixels in a defined area, for example a 100x100 pixel square about the pixel of interest

$\varepsilon$ = error margin, set to yield a predetermined error rate for falsely categorizing a pixel as not of interest.

Moreover, in a preferred embodiment, even for structures of interest, further scanning for each pixel is stopped when the measurement error for that pixel falls below a threshold. That is, when by taking into account previous surface information, information from previous scans in an integrated scan and previously scanned pixels in the same plane, the system can be certain the pixel's value has been determined to less than a predetermined tolerance, it is not scanned again.

In some situations, it is not possible to use all of the information of Eq.(1). For example, if an integrated scan is not used, the first term "R" will not be available, and any determination to avoid scanning pixels must be made entirely on the basis of correlation to a previously scanned surface or to correlated pixels already scanned in the same plane. Time duration of image formation is not an issue solely in volumetric imaging but is also relevant in mosaic imaging. For example, it may be necessary to collect a gigabyte or more of pixels in the formation of a mosaic image. In this case information from a previous plane is not available, and only the integrated scan and same scan data is available for determining areas to not scan. Finally, even in the same scan, it might not be possible to sufficiently evaluate an immediately scanned pixel, in time to make a decision on whether or not to scan a pixel that is slated to be scanned next. In each of these instances a term is dropped from equation one and $\varepsilon$ is increased, to avoid falsely deciding to not scan pixels of interest.

In a preferred embodiment, $C_R$, $C_{PSF}$, $C_{SP}$, $C_V$ and are set according to the type of material to be imaged and the orientation of internal features, which can be entered by a user. For example, if a material having a lot of long features running through the image planes is to be imaged, $C_{PSF}$ is set relatively high, and $\varepsilon$ is set relatively low. A user may introduce the material type and orientation of internal features (where appropriate), by a user interface, for example a drop down menu, that would list, for example tissue types, types of core samples. Another drop down menu permits selection of orientation.

As noted previously, in a preferred embodiment, structure of interest (for example, cell) boundaries are detected using either early scans from an integrated scan, previous surface information and/or same scan information. The areas within the boundaries are scanned further, while the areas outside the boundaries are not.

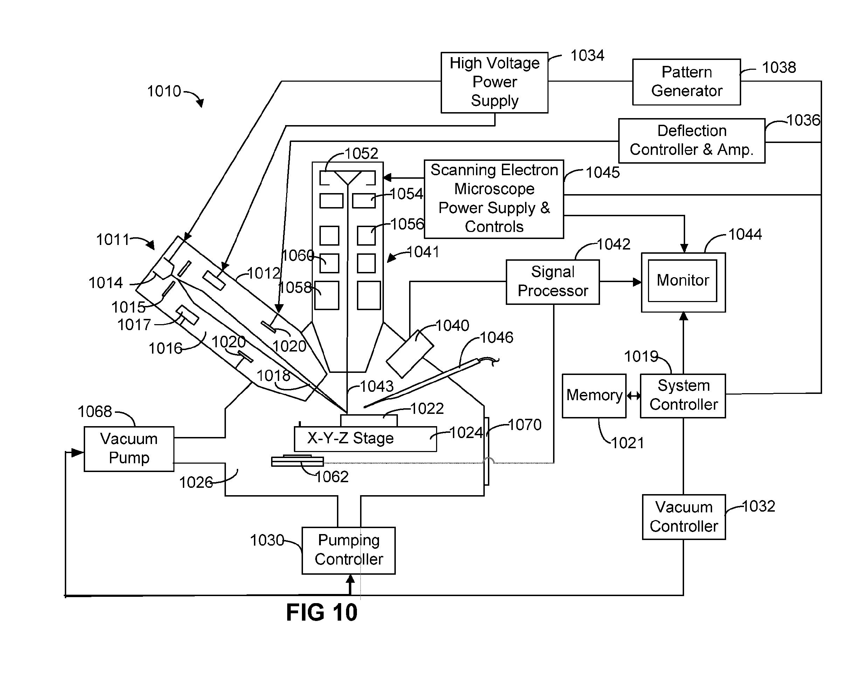

FIG. 10 shows an exemplary dual beam FIB/SEM system 1010 that could be used to implement preferred embodiments of the present invention. While reference is made to a dual beam system, aspects of the present invention are not limited to a dual beam system, but may also be implemented in other charged particle beam systems, such as single beam systems. One embodiment of the present invention utilizes a dual beam FIB/SEM system 1010 that uses an ion beam that is either normal or tilted by a few degrees to the plane of the sample surface and an electron beam having an axis that is also tilted, e.g., fifty-two degrees from the axis of ion beam. In some embodiments, the ion beam and electron beam are capable of aligning so that the fields of view of both beams are coincident to within a few microns or less. The ion beam is typically used to image and machine the work piece, and the electron beam is used primarily for imaging but can also be used for some modification of the work piece. The electron beam will typically produce an image of a higher resolution than the ion beam image, and it will not damage the viewed surface like the ion beam. The image formed by the two beams can look different, and the two beams can therefore provide more information than a single beam.

Such a dual beam system could be made from discrete components or alternatively, could be derived from a conventional device such as an Altura™ or an Expida™ system available from FEI Company of Hillsboro, Oregon. The present invention could also be implemented using other particle beam systems, including for example, single beam systems, such as FIB or SEM only systems, or dual beam systems having two FIB columns.

Focused ion beam system 1010 includes an evacuated envelope 1011 having an upper neck portion 1012 within which are located an ion source 1014 and a focusing column 1016 including extractor electrodes and an electrostatic optical system. Ion beam 1018 passes from ion source 1014 through column 1016 and between electrostatic deflection means schematically indicated at 1020 toward sample 1022 positioned on movable X-Y-Z stage 1024 within lower chamber 1026. An ion pump or other pumping system (not shown) can be employed to evacuate neck portion 1012. The chamber 1026 is evacuated with turbomolecular and mechanical pumping system 1030 under the control of vacuum controller 1032. The vacuum system provides within chamber 1026 a vacuum of between approximately $1 \times 10^{-7}$ Torr

and 5 x 10⁻⁴ Torr. If an etch assisting, an etch retarding gas, or a deposition precursor gas is used, the chamber background pressure may rise, typically to about $1 \times 10^{-5}$ Torr.

High voltage power supply 1034 is connected to ion source 1014 as well as to appropriate electrodes in focusing column 1016 for forming an ion beam 1018 and directing the same downwardly. Deflection controller and amplifier 1036, operated in accordance with a prescribed pattern provided by pattern generator 1038, is coupled to deflection plates 1020 whereby beam 1018 may be controlled to trace out a corresponding pattern on the upper surface of sample 1022. In some systems the deflection plates are placed before the final lens, as is well known in the art.

The ion source 1014 typically provides a metal ion beam of gallium, although other ion sources, such as a multicusp or other plasma ion source, can be used. The ion source 1014 typically is capable of being focused into a sub one-tenth micron wide beam at sample 1022 for either modifying the sample 1022 by ion milling, enhanced etch, material deposition, or for the purpose of imaging the sample 1022. A charged particle multiplier 1040 used for detecting secondary ion or electron emission for imaging is connected to signal processor 1042, where the signal from charged particle multiplier 1040 are amplified, converted into digital signals, and subjected to signal processing. The resulting digital signal is to display an image of sample 1022 on the monitor 1044.

A scanning electron microscope 1041, along with power supply and control unit 1045, is also provided with the FIB/SEM system 1010. An electron beam 1043 is emitted from a cathode 1052 by applying voltage between cathode 1052 and an anode 1054. Electron beam 1043 is focused to a fine spot by means of a condensing lens 1056 and an objective lens 1058. Electron beam 1043 is scanned two-dimensionally on the specimen by means of a deflection coil 1060. Operation of condensing lens 1056, objective lens 1058, and deflection coil 1060 is controlled by power supply and control unit 1045.

Electron beam 1043 can be focused onto sample 1022, which is on movable X-Y-Z stage 1024 within lower chamber 1026. Scanning electron microscope 1041 produces a finely focused electron beam 1043, which is scanned across the surface of the structure, preferably in a raster pattern. When the electrons in the electron beam 1043 strike the surface of work piece 1022, secondary electrons and backscattered electrons are emitted. Respectively, these electrons are detected by secondary electron detector 1040 or backscattered electron detector 1062. The analog signal produced either by secondary electron detector 1140 or backscattered electron detector 1062 is amplified and converted into a digital brightness value by signal processor unit 1042. The resulting digital signal can be displayed as an image of sample 1022 on the monitor 1044.

A door 1070 is opened for inserting sample 1022 onto stage 1024, which may be heated or cooled, and also for servicing an internal gas supply reservoir, if one is used. The door is interlocked so that it cannot be opened if the system is under vacuum. The high voltage power supply provides an appropriate acceleration voltage to electrodes in ion beam column 1016 for energizing and focusing ion beam 1018.

A gas delivery system 1046 extends into lower chamber 1026 for introducing and directing a gaseous vapor toward sample 1122. U.S. Pat. No. 5,851,413 to Casella et al. for "Gas Delivery Systems for Particle Beam Processing," assigned to the assignee of the present invention, describes a suitable gas delivery system 1046. Another gas delivery system is described in U.S. Pat. No. 5,435,850 to Rasmussen for a "Gas Injection System," also assigned to the assignee of the present invention. For example, iodine can be delivered to enhance etching, or a metal organic compound can be delivered to deposit a metal.

System controller 1019 controls the operations of the various parts of dual beam system 1010. Through system controller 1019, a user can cause ion beam 1018 or electron beam 1043 to be scanned in a desired manner through commands entered into a conventional user interface (not shown). System controller 1019 can also comprise computer-readable memory 1021 and may control dual beam system 1010 in accordance with data or programmed instructions stored in memory 1021. CAD data concerning the sample or stored in memory 1021 can be used to create a CAD polygon overlay or other positional data used to locate a feature of interest and alignment points or transfer fiducials as described above.

In accordance with some embodiments of the present invention, a method of using a scanning microscope to rapidly form a digital image of an area, comprises performing an initial set of scans to form a guide pixel set for said area, using said guide pixel set to identify regions representing structures of interest in said area, and performing additional scans of said regions representing structures of interest, to gather further data to further evaluate pixels in said regions, and not scanning elsewhere in said area.

In some embodiments, said scanning microscope is configured to scan an area multiple times and to combine scan results to form an integrated scan of said area, and said initial set of scans includes a first set of said multiple scans, and is used to form said guide pixel set. In some embodiments, said first set of said multiple scans includes a single scan only.

In some embodiments, said initial set of scans includes a partial scan of said area, following a scan pattern adapted to acquire structures of interest. In some embodiments, said area is a portion of a volume being scanned, by progressively removing material to reveal a new surface and each time a new surface is revealed, scanning said new surface, wherein said initial set of scans includes a scan of a previously revealed surface, within said volume.

In some embodiments, regions of interest are identified at least in part by evaluating pixel intensity. In some embodiments, regions of interest are identified at in part by evaluating regional pixel variance.

In accordance with some embodiments of the present invention, a scanning microscope assembly, adapted to perform a scan of an area, and as a part of performing said scan of said area, adapted to perform an initial set of scans to form a guide pixel set for said area, use said guide pixel set to identify regions representing structures of interest in said area, perform additional scans of said regions representing structures of interest, to gather further data to further evaluate pixels in said regions, and not scanning elsewhere in said area.

In some embodiments, said scanning microscope is configured to scan an area multiple times and to combine scan results to form an integrated scan of said area, and wherein said initial set of scans is a first set of said multiple scans, and is used to form said guide pixel set. In some embodiments, said initial set of scans includes a single scan only. In some embodiments, said initial set of scans is a partial scan of said area, following a scan pattern adapted to acquire structures of interest.

In some embodiments, said area is a portion of a volume being scanned, by progressively removing material to reveal a new surface and each time a new surface is revealed, scanning said new surface, and wherein said initial set of scans includes a scan of a previously revealed surface, within said volume. In some embodiments, regions of interest are identified at least in part by evaluating pixel intensity. In some embodiments, regions of interest are identified at least in part by evaluating regional pixel variance.

In accordance to some embodiments of the present invention, a method of forming an image of an area containing regions of interest and regions not of interest using a scanning imaging device, comprises performing a brief scan, thereby collecting less accurate information than possible, but also taking less time, using data from said brief scan to determine pixels representing regions not of interest, and performing a scan at a slower rate, but not scanning pixels determined to represent regions not of interest, thereby completing said scan more quickly.

In some embodiments, said scanning device is a scanning electron microscope. In some embodiments, said area is one area out of set of areas that are combined together to form a combined image. In some embodiments, said area is one area out of a set of areas stacked perpendicularly to the plane dimensions of said set of areas, to form a volume. In some embodiments, said area is one out of a set of areas that are mosaicked together.

In some embodiments, said quick scan is a first set of scans performed during an integrated scanning process. In some embodiments, said first set of scans includes a single scan only.

In accordance with some embodiments of the present invention, a scanning electron microscope assembly including a data entry sub-assembly, adapted to permit a user to input dimensional characteristics of structure of interest in a specimen to be scanned and wherein said dimensional characteristics affect scanning of said specimen.

In some embodiments, said dimensional characteristics effect an algorithm determining which parts of a scan are not regions of interest and are to be skipped. In some embodiments, said data entry sub-assembly includes a drop down menu.

In accordance with some embodiments of the present invention, a method of using a scanning microscope to rapidly form an image of a specimen, comprises repeatedly scanning an area of the specimen and integrating a detector signal at each pixel from the multiple scans to produce an image of the area; characterized by performing an initial scan or set of scans of a first area composed of a first set of pixels to determine a first gray level for each of the pixels in the first set; analyzing the first gray levels of the pixels in the first set to identify first regions representing structures of interest in said area, said first regions including fewer pixels than the number of pixels in the first set; and performing additional scans of said regions to determine second gray level values of only pixels of said regions, the second gray level values being determined by integrating more scans than the number of scans used to determine the first gray levels, thereby improving the accuracy of the measured gray level value of pixels in the regions while including fewer pixels in the additional scans.

In some embodiments, analyzing the first gray levels of the pixels in the first set to identify first regions includes comparing the first gray level of the pixels to thresholds, the thresholds depending on the number of integrated scans used to determine the first gray levels of the pixels. In some embodiments, analyzing the first gray levels of the pixels in the first set to identify first regions includes comparing the first gray level of the pixels to thresholds, the thresholds depending on the gray level of adjacent levels.

In some embodiments, performing additional scans comprises analyzing the second gray levels of the pixels in the second set to identify second regions representing the structures of interest in said area, said second regions being a subset of and including fewer pixels than the first set, and further comprising performing additional scans of said regions to determine third gray level values of only pixels of said second regions, the third gray level values being determined by integrating more scans than the number of scans used to determine the second gray levels.

In some embodiments, the method further comprises exposing a second area by removing from the specimen a layer of material that includes the first area; and scanning a second region of the second area, the second region comprising a subset of pixels in the second area and determined by the first regions.

In some embodiments, the method further comprises progressively removing material to reveal a new surface and each time a new surface is revealed, scanning a limited region of each new surface, the limited region being determined by the gray level of pixels in a previous scan. In some embodiments, the thresholds are determined by the gray level of neighboring pixels and the gray level of pixels at the same location in a previous layer.

In accordance with some embodiments of the present invention, a scanning charged particle beam assembly, comprises a source of charged particles; deflectors for scanning the specimen with a charged particle beam; a lens for focusing the charged particle beam onto a specimen; a detector for detecting emissions from the specimen in response to impact of the charged particles; and a computer for controlling the operation of the scanning charged particle beam assembly; **characterized in that** a computer readable memory store instruction to perform embodiments of the previously described method.

Although the present invention and its advantages have been described in detail, it should be understood that various changes, substitutions and alterations can be  made herein without departing from the invention as defined by the appended claims. Moreover, the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include such processes, machines, manufacture, compositions of matter, means, methods, or steps.

**Claims**

1.    A method of using a scanning microscope to rapidly form an image of a specimen, comprising:

repeatedly scanning an area of the specimen and integrating a detector signal at each pixel from the multiple scans to produce an image of the area;
**characterized by:**

performing an initial scan or set of scans of a first area composed of a first set of pixels to determine a first gray level value for each of the pixels in the first set;
analyzing the first gray levels of the pixels in the first set to identify first regions (10, 20) representing structures of interest in said area, said first regions including fewer pixels than the number of pixels in the first set; and
performing additional scans of said regions to determine second gray level values of only pixels of said regions, the second gray level values being determined by integrating more scans than the number of scans used to determine the first gray levels, thereby improving the accuracy of the measured gray level value of pixels in the regions while including fewer pixels in the additional scans.

2.    The method of claim 1 in which analyzing the first gray levels of the pixels in the first set to identify first regions includes comparing the first gray level of the pixels to thresholds, the thresholds depending on the number of integrated scans used to determine the first gray levels of the pixels.

3.    The method of claim 1 in which analyzing the first gray levels of the pixels in the first set to identify first regions includes comparing the first gray level of the pixels to thresholds, the thresholds depending on the gray level of adjacent pixels.

4.    The method of any of the above claims in which performing additional scans comprises analyzing the second gray levels of the pixels in the second set to identify second regions (10, 20) representing the structures of interest in said area, said second regions being a subset of and including fewer pixels than the first set, and further comprising performing additional scans of said regions to determine third gray level values of only pixels of said second regions, the third gray level values being determined by integrating more scans than the number of scans used to determine the second gray levels.

5.    The method of any of the above claims further comprising:

exposing a second area by removing from the specimen a layer of material that includes the first area; and
scanning a second region of the second area, the second region comprising a subset of pixels in the second area and determined by the first regions.

6. The method of claim 3 further comprising progressively removing material to reveal a new surface and each time a new surface is revealed, scanning a limited region of each new surface, the limited region being determined by the gray level of pixels in a previous scan.

7. The method of any of claims 1, 4, or 5 in which the thresholds are determined by the gray level of neighboring pixels and the gray level of pixels at the same location in a previous layer.

8. A scanning charged particle beam assembly, comprising:

a source of charged particles;
deflectors for scanning the specimen with a charged particle beam;
a lens for focusing the charged particle beam onto the specimen;
a detector for detecting emissions from the specimen in response to impact of the charged particles; and
a computer for controlling the operation of the scanning charged particle beam assembly;
**characterized in that** a computer readable memory stores instructions to perform the method of claim 1.

FIG. 1

FIG. 2

**FIG. 3**

**FIG. 4**

**FIG. 5**

**FIG. 6**

**FIG. 7**

FIG. 8

FIG. 9

FIG 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5851413 A, Casella
- US 5435850 A, Rasmussen